# EUROPEAN PATENT APPLICATION

(11) **EP 4 751 742 A1**
(43) Date of publication of application: **03.06.2026**
(21) Application number: 25216925.5
(22) Date of filing: 19.11.2025
(51) Int. Cl.: A61L 27/12, A61L 27/46, A61L 27/54, A61L 27/56

(54) **BIOACTIVE HYDROXYAPATITE MATERIALS MODIFIED WITH PYOMELANIN, POLYMER-CERAMIC BIOCOMPOSITES WITH PYOMELANIN HYDROXYAPATITE, METHODS OF THEIR PREPARATION AND THEIR USES**

(30) Priority: 19.11.2024 PL 45031024; 19.02.2025 PL 45124525
(71) Applicant: Uniwersytet Lodzki, 90-136 Lodz (PL); Siec Badawcza Lukasiewicz - Instytut Ceramiki i Materialow Budowlanych, 31-983 Krakow (PL); Politechnika Wroclawska, 50-370 Wroclaw (PL)
(72) Inventor: URBANIAK, Mateusz M., 99-200 Poddebice (PL); RUDNICKA, Karolina, 91-017 Lodz (PL); PLOCINSKI, PrzemysLaw, 92-761 Lodz (PL); WLODARCZYK, Marcin, 91-304 Lodz (PL); SZWED-GEORGIOU, Aleksandra, 90-553 Lodz (PL); MIKOLAJCZYK-CHMIELA, Magdalena, 91-496 Lodz (PL); BIERNAT, Monika, 02-593 Warszawa (PL); SZTERNER, Piotr, 02-761 Warszawa (PL); ANTOSIK, Agnieszka, 03-188 Warszawa (PL); GAZINSKA, Malgorzata, 55-003 Jeszkowice (PL); KROKOS, Anna, 50-504 Wroclaw (PL); TYMOWICZ-GRZYB, Paulina, 03-138 Warszawa (PL); KRUPA, Agnieszka, 90-129 Lodz (PL)
(74) Representative: Godlewski, Piotr

(57) **Abstract**

The subject of the invention is a pyomelanin-modified hydroxyapatite for the production of bioactive polymer composites, the surface of which is chemically modified by pyomelanin molecules covalently attached via a silane precursor or by pyomelanin molecules incorporated in an *in situ* reaction between the hydroxyapatite crystal lattice and three-component polymer-ceramic biocomposites with osteoconductive, immunomodulating and antibacterial properties produced in the form of flexible porous materials based on poly(glycerol adipate) (PGA) containing as a ceramic phase one of the selected pyomelanin-modified hydroxyapatites (HA): nHA-PyoM *in situ* or nHA-APTES-PyoM and calcined nanohydroxyapatite (nHA1200°C) for use, in particular in bone tissue implant materials. The subject of the invention also includes methods for obtaining hydroxyapatite and biocomposites and their use for filling bone defects and for bone regeneration, and more specifically for dental, veterinary and orthopedic implantations, including bone oncology.

## Description

The present invention relates to bioactive hydroxyapatite materials modified with pyomelanin and ternary polymer-ceramic biocomposites with osteoconductive, immunomodulatory and antimicrobial properties for use, in particular in bone tissue implant materials. The invention also relates to methods of obtaining them and their applications. More specifically, the invention relates to synthetic, bioactive hydroxyapatite materials modified with pyomelanin isolated from the *Pseudomonas aeruginosa* bacterium and polymer-ceramic biocomposites, which are produced in the form of flexible porous materials based on poly(glycerol adipate) (PGA) containing as a ceramic phase one of the hydroxyapatites (HA) modified with pyomelanin (PyoM): nHA-PyoM*_{in situ}* or nHA-APTES-PyoM and calcined nanohydroxyapatite (nHA1200°C).

Bone is mineralized connective tissue responsible for key physiological functions and processes in the body, such as body shape and movement, support and protection of organs and soft tissues, synthetic functions related to erythropoiesis and lymphopoiesis, and metabolic functions including mineral storage, regulation of calcium and phosphate metabolism, and maintenance of hormonal homeostasis. Bone damage resulting from diseases such as osteoporosis, cancer or bacterial infections, or injuries resulting from accidents, is one of the most common causes of hospitalization, which often requires implantation of bone substitute materials. Traditional materials for repairing bone defects mainly include the implantation of autogenous or xenogeneic bone, decalcified bone matrix, bioceramics or metal-derived materials. The indicated materials, apart from autologous bone, are far from ideal in terms of properties essential for proper bone tissue reconstruction, including biocompatibility, biodegradability, porosity of the spatial structure, osteoinductivity and support of osteogenesis.

One of the modern materials used in bone tissue regenerative medicine characterized by a wide range of biocompatibility is hydroxyapatite (HA). Synthetic HA [Ca₁₀(PO₄)₆(OH)₂] is a calcium orthophosphate with a calcium/phosphorus molar ratio (Ca/P) of 1.667. HA occurs in several different morphological forms of crystals of varying sizes. Obtaining different morphological forms of crystalline HA depends on the synthesis method used, its conditions and the substrates that are the source of calcium ions (Ca) and phosphate ions (PO₄³⁻). HA can be obtained from natural sources or by appropriate chemical syntheses using wet, hydrothermal, dry, ultrasonic or microwave methods. The use of chemical syntheses compared to obtaining HA from natural sources provides many advantages, including product purity, repeatability of structure and the ability to control biological activity.

In recent years, the development of hydroxyapatite ceramics has focused on achieving the highest possible similarity of the hydroxyapatite material to natural apatites, which are mineral components found in bone tissue and are responsible for the mechanical properties of bones. Moreover, the possibility of modifying calcium phosphate ceramics with active biological compounds enables obtaining a higher level of biocompatibility and regenerative potential, as well as controlled immunomodulatory, antibacterial or osteoconductive properties.

Pyomelanin (PyoM) is a negatively charged black-brown extracellular polymer of homogentisic acid that is secreted by the bacterium *Pseudomonas aeruginosa.* There are two known forms of this bacterial dye: soluble (PyoMₛₒₗ) and insoluble in water (PyoMᵢₙₛₒₗ), obtained depending on the isolation procedure from bacterial cell culture supernatants. The chemical structure of PyoM determines its oxidizing and reducing properties, which are responsible for basic activities related to the neutralization of reactive oxygen species, protection against ultraviolet light, electron transport and binding of metal ions. Due to the verified biosafety of PyoM at the level of *in vitro* and *in vivo* and numerous biological activities, this dye can be used in medicine and materials engineering, in particular for the production of modified hydroxyapatite ceramics and polymer-ceramic biocomposites.

PyoM has antibacterial activity against many species of pathogenic bacteria, including: *Staphylococcus aureus, Escherichia coli, Vibrio parahaemolyticus, Klebsiella pneumoniae, Enterococcus faecalis, Listeria monocytogenes* and *Shigella dysenteriae* and supports cell regeneration by neutralizing reactive oxygen species and stimulating immune system cells. Moreover, PyoM stimulates the activation of nuclear factor κB (NF-κB, *nuclear factor kappa-light-chain-enhancer of activated B cells*), which initiates the processes of tissue reconstruction and regeneration, including bone tissue. NF-κB also induces the expression of pro-proliferative genes responsible for promoting cellular repair processes and may also exert an inhibitory effect on the production of pro-inflammatory cytokines to alleviate inflammation. Research also indicates a broad role of this transcription factor in various cellular processes, including the initiation and control of the inflammatory process in bone tissue, proliferation, differentiation, maturation and migration of osteoblasts, osteoclasts and immune cells.

The publication *"Designing bioinspired multifunctional nanoplatforms to support wound healing and skin regeneration: Mg-hydroxyapatite meets melanins"* discloses a method for the synthesis of nano-hydroxyapatite doped with magnesium (MgHA) and synthetic melanin resulting from the polymerization of 5,6-dihydroxyindole-2-carboxylic acid (DHICA) or dopamine, and its biological activity in the context of stimulating the migration of BALB/3T3 fibroblast cells in a simulated wound environment. The method according to the publication enables obtaining the product in a multi-stage process, and the aqueous solutions are subjected to precipitation, washing, sonication and drying, and then coating MgHA with melanin by dropwise addition of DHICA in ethanol or a dopamine solution in ethanol. The MgHA/melanin precursor molar ratio was 8. Ca(OH)₂ was used as a precursor of calcium ions, was used as a precursor of phosphate ions, and magnesium ions were derived from MgCl₂. Dopamine and DHICA have been exploited as precursors for synthetic melanin.

Patent application no. P 443565 describes a method for obtaining a chitosan-based composite, comprising a stage of biotechnological preparation of an active dye, a stage of preparation of metallic nanoparticles and the production of a liquid nanocomposite, characterized in that an active dye, i.e. pyomelanin, is added to 30 cm³ of a 2% (w/v) chitosan solution to a final concentration of 200 µg/cm³ and 3 cm of a colloidal solution of silver nanoparticles with a concentration of 400 µg/cm³, optionally, hydroxyapatite is added to the mixture in an amount of 1% by weight relative to the volume of the mixture and mixed at room temperature until a homogeneous suspension is obtained. The subject of the invention is also a nanocomposite obtained by such a method and the use of the nanocomposite for the regeneration of bone tissue defects.

US patent application US/2020/155726 A1 presents a biomaterial composite consisting of silk fibroin and melanin intended for use in the treatment of rheumatic disease, neurodegenerative diseases, cardiovascular diseases and/or wound healing.

Chinese patent application CN111825892A describes a melanin-graphene modified latex composite material for industrial applications.

Despite many known solutions for the synthesis and modification of HA, there is still a search for innovative hydroxyapatite materials with documented biological properties that can be used in regenerative medicine of bone tissue, as well as biocomposites based on the above-mentioned materials.

The disadvantages of known polymer-ceramic biocomposites for use as filling of bone defects and for bone regeneration are:
- lack of effective osteoinductive, osteoconductive, immunomodulatory and antibacterial effects,
- inappropriate degree of porosity ensuring poor level of cell adhesion,
- stiffness and brittleness, and
- lack of tolerance in matching the shape to the dimensions of the bone defect.

The aim of the invention was therefore to obtain new bioactive hydroxyapatite materials modified with pyomelanin, as well as to develop polymer-ceramic biocomposites based on these hydroxypatites, which would be porous elastic materials, and therefore characterized by elasticity and reversibility of deformations, for use as filling of bone defects and for bone regeneration, so that they would have proven bioactive properties in terms of supporting osteoblast proliferation, immunomodulating, osteoinductive and osteoconductive properties, as well as antibacterial activity.

The subject of the invention is a pyomelanin-modified hydroxyapatite, characterized in that the hydroxyapatite surface is chemically modified by pyomelanin molecules covalently attached via a silane precursor or by pyomelanin molecules embedded between the hydroxyapatite crystal lattice in an *in situ* reaction.

Another subject of the invention is a method for producing hydroxyapatite modified with covalently attached pyomelanin, characterized in that hydroxyapatite particles are subjected to a two-stage chemical modification, so that in the first stage the particles are reacted with a silane precursor, and then the product of the first stage of modification is reacted with pyomelanin in a solvent.

Preferably, the method for producing hydroxyapatite modified with covalently attached pyomelanin is carried out in such a way that the first stage of modification of hydroxyapatite particles is carried out in a suspension of hydroxyapatite particles at a concentration of 0.1 to 3 g per 5 mL of solvent at a temperature of 100°C to 150°C, during 24 hours, at a mass ratio of hydroxyapatite particles to the volume of the silane precursor of 1:0.5, and the pyomelanin modification reaction in the second stage is carried out using 1-ethyl-3-(3-dimethylaminopropyl)carbodiimide hydrochloride (EDC) as a coupling agent and sodium salt of N-hydroxysulfosuccinimide (sulo-NHS) as an agent increasing the reaction efficiency.

Preferably, the solvent is anhydrous toluene, the silane precursor is 3-aminopropyltriethoxylate (APTES) and the pyomelanin is bacterial pyomelanin in a soluble form (PyoMₛₒₗ) or insoluble in water (PyoMᵢₙₛₒₗ).

Preferably, the hydroxyapatite particles are subjected to sonication in the first stage and the reaction with the EDC coupling agent is carried out in a 4-morpholinoethanesulfonic acid buffer at pH 6.

Another subject of the invention is a method for producing hydroxyapatite, in which pyomelanin molecules are incorporated into the hydroxyapatite crystal lattice in an *in situ* reaction, characterized in that:
- to the aqueous reaction solution of Ca(OH)₂ pyomelanin is added and then an aqueous solution of orthophosphoric acid is dosed in divided doses,
- the reaction is carried out at a constant temperature in the range of 25°C to 75°C for 2 to 48 hours, at a constant pH of the solution in the range of 9 to 11, under continuous stirring at a speed of 450 rpm to 1600 rpm, and
- the orthophosphoric acid solution is dosed into the reaction mixture over a period of 35-45 minutes, maintaining the equilibrium state of the substrates for no less than 3.5 hours until exhaustion and precipitation of a precipitate with nanometric-sized crystalline particles of hydroxyapatite modified with pyomelanin, after which
- the sediment is purified by repeated centrifugation alternating with washing with deionized water until a neutral pH is obtained,
- it is dried for 24 to 48 hours at a temperature not higher than 90°C.

Yet another subject of the invention is a method for producing hydroxyapatite, in which pyomelanin molecules are incorporated into the hydroxyapatite crystal lattice in an *in situ* reaction, characterized in that:
- to the aqueous reaction solution of Ca(OH)₂ the aqueous solution of orthophosphoric acid is dosed in divided doses in the first stage and then
- pyomelanin dissolved or suspended in an ammonia solution is added, wherein the reaction is carried out at a constant temperature in the range of 25°C to 80°C for a time of 2 to 72 hours, at a constant pH of the solution in the range of 9 to 11, and under continuous stirring at a speed of 450 rpm to 1600 rpm, and
- the orthophosphoric acid solution is dosed into the reaction mixture over a period of 35-45 minutes, maintaining the equilibrium state of the substrates for no less than 3.5 hours until exhaustion and precipitation of a precipitate with nanometric-sized crystalline particles of hydroxyapatite modified with pyomelanin, after which
- the sediment is purified by repeated centrifugation alternating with washing with deionized water until a neutral pH is obtained,
- it is dried for 24 to 72 hours at a temperature not higher than 90°C.

Preferably, the molar ratio of calcium atoms derived from Ca(OH)₂ to phosphorus atoms in H₃PO₄ is Ca/P=1.67, and per 0.05 moles Ca(OH)₂ there is from 10 to 50 mg of pyomelanin, preferably 25 mg of pyomelanin.

Another subject of the invention is a polymer-ceramic biocomposite based on hydroxyapatite, characterized in that it contains poly(glycerol adipate) (PGA) modified with L-lysine diisocyanate ethyl ester, and as a ceramic phase nanohydroxyapatite calcined at 1200°C (nHA1200°C) and hydroxyapatite (HA) modified with pyomelanin (PyoM), obtained by the methods described above.

Preferably, the pyomelanin-modified hydroxyapatite (HA) (PyoM) is hydroxyapatite with pyomelanin molecules covalently attached via a silane precursor or pyomelanin molecules embedded between the hydroxyapatite crystal lattice by an *in situ* reaction.

Preferably, the biocomposite consists of poly(glycerol adipate) chemically cross-linked with **L-**lysine diisocyanate ethyl ester in an amount of 56.65 wt.% based on the total weight of the composite, nanohydroxyapatite calcined at 1200°C in an amount of 42.50 wt.% based on the total weight of the composite, and hydroxyapatite with pyomelanin molecules covalently attached via a silane precursor or pyomelanin molecules incorporated in an *in situ* reaction between the hydroxyapatite crystal lattice in an amount of 0.85 wt.% based on the total weight of the composite, wherein the hydroxyapatite with pyomelanin molecules covalently attached via a silane precursor contains pyomelanin in an amount of 2.58 wt.%. in relation to the total hydroxyapatite modified with pyomelanin, while hydroxyapatite with pyomelanin molecules incorporated in the *in situ* reaction between the hydroxyapatite crystal lattice contains pyomelanin in the amount of 4.29% by weight in relation to the total hydroxyapatite modified with pyomelanin.

The biocomposite advantageously supports osteoblast proliferation and has immunomodulatory, osteoinductive, osteoconductive and antibacterial properties.

Yet another subject of the invention is a method for obtaining a biocomposite with osteoinductive, osteoconductive, immunomodulating and antibacterial properties based on hydroxyapatite and chemically modified poly(glycerol adipate), characterized in that:
- in the first stage, chemical modification of the poly(glycerol adipate) prepolymer obtained by a known method is carried out with L-lysine diisocyanate ethyl ester in anhydrous dioxane at a temperature of 20-80°C for 2 to 8 hours;
- then a mixture of previously obtained particles of calcined hydroxyapatite and hydroxyapatite covalently modified with pyomelanin via a silane precursor or particles of calcined hydroxyapatite and hydroxyapatite with pyomelanin particles incorporated in the *in situ* reaction between the crystal lattice of hydroxyapatite and NaCl salt is prepared;
- the mixture is poured with a solution of poly(glycerol adipate) modified with L-lysine diisocyanate ethyl ester in dioxane;

- the resulting dispersion system is frozen at a temperature of -10 to -40 for 12 to 48 hours, preferably -20°C for 24 hours;
- it is then freeze-dried for 12 to 48 hours, preferably 24 hours; after which
- it is crosslinked at temperatures from 30 to 90°C for 12 to 72 hours, preferably 60°C and for 48 hours, and then
- NaCl salt is washed out and dried at 30-80°C for 12-72 hours, preferably 50°C and 24 hours.

Preferably, the crosslinking agent used in the first stage - L-lysine diisocyanate ethyl ester is used in the amount of 2.64 mmol per 1 g of poly(glycerol adipate), the NaCl salt in the mixture constitutes 90.75% by weight in relation to the weight of the ceramic particle mixture, the chemical modification of poly(glycerol adipate) is carried out at 50°C for 5 hours in a nitrogen atmosphere and sodium chloride with a grain size of 400 to 500 µm is used as the blowing agent.

The subject of the invention is also the use of hydroxyapatite and the biocomposite defined above for filling bone defects, bone regeneration and dental, veterinary and orthopedic implantations, including bone oncology.

Pyomelanin-modified hydroxyapatites obtained by the described methods are used in bone tissue implantation materials as fillers of polymer-ceramic composites intended for dental, orthopedic and veterinary implantations, exhibiting immunomodulatory properties. Indicated applications include the development of implants that not only support osseointegration but also minimize the risk of inflammatory reactions, which is crucial for long-term effectiveness and reduced postoperative complications. Possible areas of application of the described hydroxyapatites include **veterinary medicine** (especially in the treatment of bone injuries in pets such as dogs, cats, exotic and breeding species - endangered species or racehorses, where quick, effective solutions are required and their cost is not a market barrier); **dentistry** (as a component of fillers in polymer-ceramic composites used in dental implants, ensuring faster healing, better bonding to the bone and reduced inflammatory response at the implant site; additionally, their antibacterial properties resulting from the presence of pyomelanin may reduce the risk of infection, which is a common problem in dental procedures), **orthopedics** (in the production of bone implants used in the reconstruction of large bone defects, e.g. in hip or knee replacement surgeries or in the treatment of post-traumatic injuries, allowing for increased treatment effectiveness by accelerating the osseointegration process and reducing inflammation around implants) and **bone oncology** (in the case of patients after resection of bone tumors, modified hydroxyapatites can be used as a regenerative material, supporting bone reconstruction in a safe manner).

New osteoinductive, osteoconductive, immunomodulating and antibacterial elastomeric polymer-ceramic composites with confirmed cytocompatibility towards normative and bone cells, based on poly(glycerol adipate) chemically cross-linked with L-lysine diisocyanate ethyl ester and nanohydroxyapatite calcined at 1200°C and one of the hydroxyapatites modified with pyomelanin: nHA-PyoM*_{in situ}* or nHA-APTES-PyoM according to the invention, are used as an implantation material for filling bone defects and regenerating bone tissue.

As for the amount of individual components in the biocomposite: poly(glycerol adipate) chemically cross-linked with L-lysine diisocyanate ethyl ester is present in the amount of 56.65% by weight of the total weight of the composite, nanohydroxyapatite particles calcined at 1200°C are present in the amount of 42.50% by weight of the total weight of the composite, and hydroxyapatite particles with pyomelanin covalently attached via a silane precursor or hydroxyapatite with pyomelanin incorporated in an *in situ* reaction between the hydroxyapatite crystal lattice are present in the amount of 0.85% by weight of the total weight of the composite.

The composite contains as a filler hydroxyapatite modified with pyomelanin in the form of: hydroxyapatite particles with pyomelanin molecules covalently attached via a silane precursor with a pyomelanin content of 2.58% by weight in relation to the total hydroxyapatite modified with pyomelanin, or hydroxyapatite particles with pyomelanin molecules incorporated in an *in situ* reaction between the hydroxyapatite crystal lattice with a pyomelanin content of 4.29% by weight in relation to the total hydroxyapatite modified with pyomelanin.

The elastomer composites of the invention in the form of elastic porous materials are obtained by the following stages:
1. Synthesis of PyoM-modified nanohydroxyapatite: nHA-PyoM *in situ* according to the described methods I-II or HA-APTES-PyoM.
2. Synthesis of poly(glycerol adipate) prepolymer by a known method via an enzymatically catalyzed polytransesterification reaction of divinyl adipate and glycerol.
3. Modification of poly(glycerol adipate) with L-lysine diisocyanate ethyl ester.
4. Preparation of a mixture of NaCl salts and nanohydroxyapatite calcined at 1200°C and one of the hydroxyapatites modified with PyoM: nHA-PyoM*_{in situ}* or nHA-APTES-PyoM
5. Dispersion of nanohydroxyapatite calcined at 1200°C and one of the hydroxyapatites modified with PyoM: nHA-PyoM*_{in situ}* or nHA-APTES-PyoM in poly(glycerol adipate) modified with L-lysine diisocyanate ethyl ester.
6. Freeze-drying of dispersions.
7. Chemical and thermal crosslinking of the polymer matrix of composites.
8. Washing out the blowing agent in the form of NaCl salt.

The invention is shown in the drawings, in which:

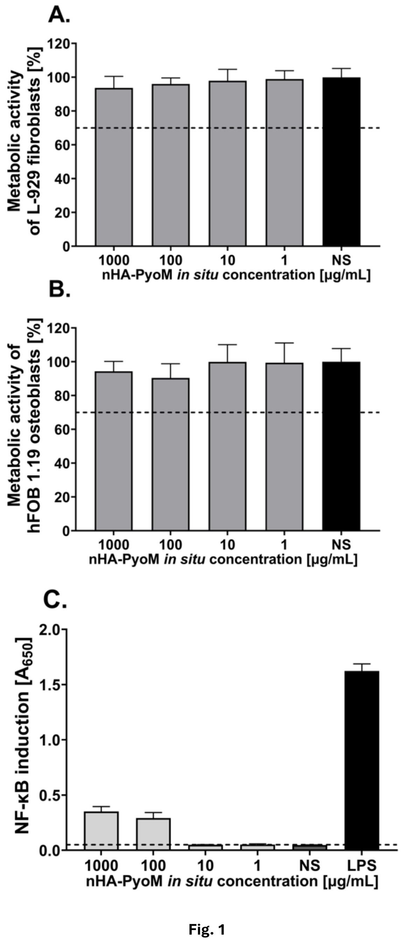

**Fig. 1** presents the biological activity of the product HA-PyoMₛₒₗ *in situ* in terms of cytocompatibility: (A.) relative to reference L-929 fibroblasts and (B.) hFOB 1.19 osteoblasts and (C.) activation of the NF-κB pathway.

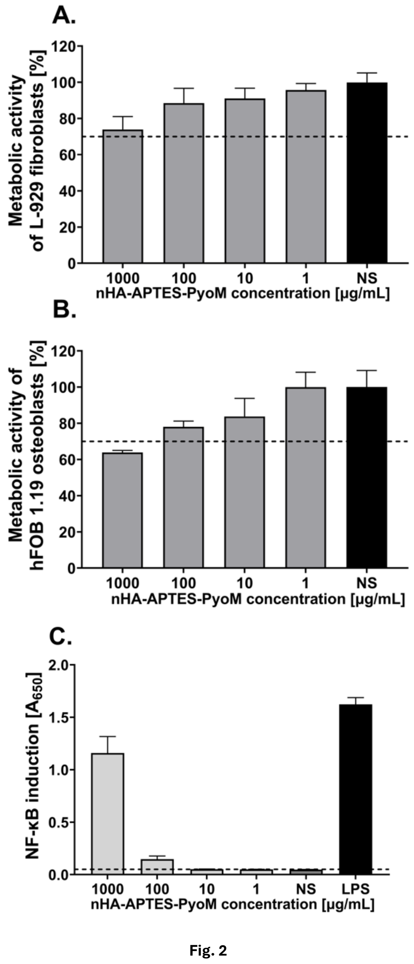

**Fig. 2** presents the biological activity of the HA-APTES-PyoM product in terms of cytocompatibility: (A.) relative to reference L-929 fibroblasts and (B.) hFOB 1.19 osteoblasts and (C.) activation of the NF-κB pathway.
**Fig. 3** presents graphs of biological activity of the PGA_nHA1200_nHA-PyoM*_{in situ}* biocomposite in terms of cytocompatibility (A) against reference L-929 fibroblasts, (B) hFOB 1.19 osteoblasts and (C) activation of the NF-κB pathway.
**Fig. 4** presents a graph of the biological activity of the PGA_nHA1200_nHA-APTES-PyoM biocomposite in terms of cytocompatibility (A) against reference L-929 fibroblasts, (B) hFOB 1.19 osteoblasts and (C) activation of the NF-κB pathway.
**Fig. 5** presents the graph of biological activity of the PGA_nHA1200_nHA-PyoM biocomposite*_{in situ}* in terms of proliferation (A), ALP activity (B), secretion: OC (C), IL-6 (D), IL-10 (E) i TNF-α (F).
**Fig. 6** presents a graph of the biological activity of the PGA_nHA1200_nHA-APTES-PyoM biocomposite presented in Fig. 4. in terms of proliferation (A), ALP activity (B), secretion: OC (C), IL-6 (D), IL-10 (E) i TNF-α (F).
**Fig. 7** presents the graph of biological activity of the PGA_nHA1200_nHA-PyoM*_{in situ}* biocomposite in terms of limiting the metabolic activity of the reference strain *Staphylococcus aureus* ATC 29213 (A) and a clinical methicillin-resistant strain isolated from infected bone tissue *Staphylococcus aureus* MRSA (B).
**Fig. 8** presents a graph of the biological activity of the PGA_nHA1200_nHA-APTES-PyoM biocomposite in terms of limiting the metabolic activity of the reference strain *Staphylococcus aureus* ATC 29213 (A) and a clinical methicillin-resistant strain isolated from infected bone tissue *Staphylococcus aureus* MRSA (B).
**Fig. 9** presents the proliferation of lymphocytes isolated from the spleens and lymph nodes of mice that were subcutaneously administered aqueous extracts obtained after incubation with the PGA _nHA1200_nHA-PyoM*_{in situ}* composite: (A) - spleen, (B) - lymph nodes.
**Fig. 10** presents the proliferation of lymphocytes isolated from the spleens and lymph nodes of mice that received subcutaneously aqueous extracts obtained after incubation with the PGA_nHA1200_nHA-APTES-PyoM composite: (A) - spleen, (B) - lymph nodes.

The subject of the invention is presented in the following embodiment examples, which are not of a limiting nature:

### Example 1

### Synthesis of pyomelanin-modified hydroxyapatite PyoMₛₒₗ in situ (method I)

Hydroxyapatite modified HA-PyoMₛₒₗ *in situ* obtained by method I using pyomelanin PyoMₛₒₗ, in which pyomelanin was added in a solution of Ca(OH)₂ at the beginning of synthesis, in order to incorporate its molecules into the hydroxyapatite crystal lattice. 40 mL of demineralized water was placed in a round-bottom flask equipped with a reflux condenser and 3.705 g of Ca(OH)₂ was added, which was dissolved under magnetic stirring (650 rpm) at 75°C. 1 mL of Ca(OH)₂ solution was taken from the flask into a separate vessel (beaker), 25 mg of pyomelanin PyoMsol was added to it and dissolved, and then the entire content of the beaker was poured into the flask with the Ca(OH)₂ solution. A solution of orthophosphoric acid was prepared in a measuring cylinder by measuring 2.042 mL of orthophosphoric acid and topping up to 10 mL with demineralized water. Then the orthophosphoric acid solution was transferred from the cylinder to the dropping funnel and the whole was added dropwise to the flask containing Ca(OH)₂ and pyomelanin for 45 minutes, maintaining the temperature at 70°C. 25% ammonia solution was then added in sufficient quantity to adjust the pH of the solution to 11. The reaction was carried out for 3 hours, maintaining the pH of the solution at 11. After the reaction was completed, the mixture in the flask was left for 3 hours. After this time, the contents of the flask were centrifuged (3700 rpm, 5 min), washed with demineralized water until a neutral pH is obtained, and the purified product was dried at 90°C for 24 hours, then ground to obtain a homogeneous powder.

The phase composition of the obtained product was determined by X-ray diffraction (XRD), the chemical structure was confirmed by Fourier transform infrared spectroscopy (FTIR), and the specific surface area was determined by the BET method.

The resulting product is a light brown powder. XRD analysis confirmed the production of hydroxyapatite (COD-9002213). SEM and BET analyses showed that a nanometric product was obtained in the form of agglomerated grains with a specific surface area of S_{BET} = 92.2495 ± 0.1503 m²/g. The FTIR spectrum of HA-PyoMₛₒₗ *in situ* shows the absorption bands characteristic for HA at 3568 cm⁻¹, indicating the vibrations of hydroxyl groups on the HA surface. Bands at 3442 cm⁻¹ and 1637 cm⁻¹ are related to the presence of water, with the band at 3442 cm⁻¹ is broadened in the range 2929-2859 cm⁻¹, which contains characteristic bands derived from pyomelanin. Characteristic bands in the range 1468-1419 cm⁻¹ and at 874 cm⁻¹ are responsible for C-O bonds in carbonate groups CO₃²⁻. The bands corresponding to the vibrational vibrations associated with phosphate ions were identified at 962 cm⁻¹, 471cm⁻¹, 1093 1032 cm⁻¹, 604 565 cm⁻¹.

Content of PyoMₛₒₗ in the structure of particles of HA-PyoMₛₒₗ *in situ* calculated based on thermogravimetric analysis from the difference between the total mass loss in the range of 25 - 900°C for HA-PyoMₛₒₗ *in situ* and for pure HA and it is 4.29% by weight. The thermal stability of the product corresponding to the temperature of 5% mass loss is 181.5°C, which is higher than the thermal stability of PyoMₛₒₗ (T-5%=81.0°C).

Cytocompatibility HA-PyoMₛₒₗ *in situ* tested in the MTT tetrazolium salt reduction test according to ISO 10993-5:2009 on the *in vitro* model and activation of the nuclear factor NF-κB in the human monocyte reporter model THP1-Blue^{™} NF-κB Cells. HA-PyoMₛₒₗ *in situ* demonstrated biological safety according to the ISO 10993-5:2009 standard for both tested cell lines in the concentration range of 1-1000 µg/mL and activated the NF-κB pathway in the concentration range of 100-1000 µg/mL. Biological studies have shown that HA-PyoMₛₒₗ *in situ* is cytocompatible and has beneficial immunomodulatory properties in the context of activating the NF-κB pathway.

### Example 2

### Synthesis of pyomelanin-modified hydroxyapatite PyoMₛₒₗ in situ (method II)

Hydroxyapatite modified HA-PyoMₛₒₗ *in situ* was obtained by method II using pyomelanin PyoMₛₒₗ, in which pyomelanin was added in an ammonia solution at the stage of changing the pH of the reaction medium in order to incorporate its molecules into the hydroxyapatite crystal lattice. In a round-bottom flask equipped with a reflux condenser, 40 mL of demineralized water was placed and 3.705 g of Ca(OH)₂ was added, which was dissolved under magnetic stirring (700 rpm) at 80°C. In a measuring cylinder, an orthophosphoric acid solution was prepared by measuring 2.042 mL of orthophosphoric acid and topping up to 10 mL with demineralized water. Then the orthophosphoric acid solution was transferred from the cylinder to the dropping funnel and the whole was added dropwise to the flask containing Ca(OH)₂ for 35 minutes, maintaining the temperature at 75°C. In a separate beaker, 25 mg of pyomelanin PyoMₛₒₗ was dissolved in 3 mL of 25% ammonia solution and the contents of the beaker were added to the flask. Sufficient 25% ammonia solution was then added to the flask to bring the pH of the solution to 11. The reaction was carried out for 3 hours, maintaining the pH of the solution at 11. After the reaction was completed, the mixture in the flask was left for 3 hours. After this time, the contents of the flask were centrifuged (3700 rpm, 3 min), washed with demineralized water until a neutral pH is obtained, and the purified product was dried at 90°C for 72 hours and ground.

The resulting product is a light brown powder. XRD analysis confirmed the production of hydroxyapatite (COD-9002213). SEM and BET analyses showed that a nanometric product was obtained in the form of agglomerated grains with a specific surface area of S_{BET} = 84.8974 ± 0.1098 m²/g. The FTIR spectrum of HA-PyoMₛₒₗ *in situ* shows the absorption bands characteristic for HA at 3568 cm⁻¹, indicating the vibrations of hydroxyl groups on the HA surface. Bands at 3423 cm⁻¹ and 1632 cm⁻¹ are related to the presence of water, with the band at 3423 cm⁻¹ is broadened in the range of 2929-2859 cm⁻¹, which contains characteristic bands derived from pyomelanin. Characteristic bands in the range 1468-1421 cm⁻¹ and at 876 cm⁻¹ are responsible for C-O bonds in carbonate groups CO₃²⁻. The bands corresponding to the vibrational vibrations associated with phosphate ions were identified at 962 cm⁻¹, 471cm⁻¹, 1093 1032 cm⁻¹, 604 565 cm⁻¹.

### Example 3

### Synthesis of pyomelanin-modified hydroxyapatite PyoMᵢₙₛₒₗ in situ (method I)

Hydroxyapatite modified HA-PyoMᵢₙₛₒₗ *in situ* was obtained by method I using pyomelanin PyoMᵢₙₛₒₗ, in which pyomelanin was added suspended in a solution of Ca(OH)₂ at the beginning of the reaction, in order to incorporate its molecules into the hydroxyapatite crystal lattice. 40 mL of demineralized water was placed in a round-bottom flask equipped with a reflux condenser and 3.705 g of Ca(OH)₂ was added, which was dissolved under magnetic stirring (850 rpm) at 65°C. 1 mL of Ca(OH)₂ solution was taken from the flask and placed in a separate vessel (beaker), 25 mg of PyoMᵢₙₛₒₗ pyomelanin was added to it, and then the entire contents of the beaker were poured into the flask. A solution of orthophosphoric acid was prepared in a measuring cylinder by measuring 2.042 mL of orthophosphoric acid and topping up to 10 mL with demineralized water. Then the orthophosphoric acid solution was transferred from the cylinder to the dropping funnel and the whole was added dropwise to the flask containing Ca(OH)₂ and pyomelanin for 35 minutes, maintaining the temperature at 65°C. 25% ammonia solution was then added in sufficient quantity to adjust the pH of the solution to 11. The reaction was carried out for 3 hours, maintaining the pH of the solution at 11. After the reaction was completed, the mixture in the flask was left for 3.5 hours. After this time, the contents of the beaker were centrifuged (3700 rpm, 2 min), washed with demineralized water until a neutral pH is obtained, and the purified product was dried at 90°C for 48 hours and ground.

The resulting product is a light brown powder. XRD analysis confirmed the production of hydroxyapatite (COD-9002213). SEM and BET analyses showed that a nanometric product was obtained in the form of agglomerated grains with a specific surface area of S_{BET} = 97, 5718 ± 0.1571 m²/g. The FTIR spectrum of HA-PyoMᵢₙₛₒₗ *in situ* shows the absorption bands characteristic for HA at 3568 cm⁻¹, indicating the vibrations of hydroxyl groups on the HA surface. Bands at 3448 cm⁻¹ and 1637 cm⁻¹ are related to the presence of water, with the band at 3448 cm⁻¹ is broadened in the range 2929-2859 cm⁻¹, which contains characteristic bands derived from pyomelanin. Characteristic bands in the range 1471-1421 cm⁻¹ and at 874 cm⁻¹ are responsible for C-O bonds in carbonate groups CO₃²⁻. The bands corresponding to the vibrational vibrations associated with phosphate ions were identified at 962 cm⁻¹, 471cm⁻¹, 1092 1032 cm⁻¹, 602 565 cm⁻¹.

### Example 4

### Synthesis of pyomelanin-modified hydroxyapatite PyoMᵢₙₛₒₗ in situ (method II)

Hydroxyapatite modified HA-PyoMᵢₙₛₒₗ *in situ* was obtained by method II using pyomelanin PyoMᵢₙₛₒₗ, in which pyomelanin was added suspended in an ammonia solution at the stage of changing the pH of the reaction medium in order to incorporate its molecules into the hydroxyapatite crystal lattice. In a round-bottom flask equipped with a reflux condenser, 40 mL of demineralized water was placed and 3.705 g of Ca(OH)₂ was added, which was dissolved under magnetic stirring (750 rpm) at 75°C. In a measuring cylinder, an orthophosphoric acid solution was prepared by measuring 2.042 mL of orthophosphoric acid and topping up to 10 mL with demineralized water. Then the orthophosphoric acid solution was transferred from the cylinder to the dropping funnel and the whole was added dropwise to the flask containing Ca(OH)₂ over 45 minutes, maintaining the temperature at 70°C. In a separate vessel (beaker), 25 mg of pyomelanin PyoMᵢₙₛₒₗ was suspended in 3 mL of 25% ammonia solution and the whole was added to the flask. Sufficient 25% ammonia solution was then added to the flask to bring the pH of the solution to 11. The reaction was carried out for 3 hours, maintaining the pH of the solution at 11. After the reaction was completed, the mixture in the flask was left for 3.5 hours. After this time, the contents of the flask were centrifuged (3700 rpm, 3 min), washed with demineralized water until a neutral pH is obtained, and the purified product was dried at 90°C for 36 hours and ground.

The resulting product is a light brown powder. XRD analysis confirmed the production of hydroxyapatite (COD-9002213). SEM and BET analyses showed that a nanometric product was obtained in the form of agglomerated grains with a specific surface area of S_{BET} = 881.0566 ± 0.1047 m²/g. The FTIR spectrum of HA-PyoMₛₒₗ *in situ* shows the absorption bands characteristic for HA at 3568 cm⁻¹, indicating the vibrations of hydroxyl groups on the HA surface. Bands at 3439 cm⁻¹ and 1630 cm⁻¹ are related to the presence of water, with the band at 3439 cm⁻¹ is broadened in the range of 2929-2859 cm⁻¹, which contains characteristic bands derived from pyomelanin. Characteristic bands in the range 1479-1421 cm⁻¹ and at 876 cm⁻¹ are responsible for C-O bonds in carbonate groups CO₃²⁻. The bands corresponding to the vibrational vibrations associated with phosphate ions were identified at 962 cm⁻¹, 471cm⁻¹, 1092 1032 cm⁻¹, 604 563 cm⁻¹.

### Example 5

### Synthesis of pyomelanin-modified hydroxyapatite PyoMₛₒₗ using a silane precursor [HA-APTES-PyoM]

The hydroxyapatite (HA) intended for modification was obtained in accordance with patent application P.442461. In a round-bottom flask 1 equipped with a reflux condenser, 3.705 g of Ca(OH)₂ was prepared, 40 mL of deionized water was added and placed on a magnetic stirrer operating at 450 rpm for 20 min at 50°C until a homogeneous suspension was obtained. In a round-bottomed flask 2 equipped with a reflux condenser, 2.042 mL of orthophosphoric acid (H₃PO₄) and diluted with deionized water to 10 mL. The acid solution prepared in this way was added dropwise over 45 minutes to the calcium hydroxide mixture placed on a magnetic stirrer operating with continuous stirring at a speed of 600 rpm. During the addition of orthophosphoric acid, pH=11 was maintained by adding ammonia water in a 25% solution. The solution was stirred for a further 2 hours while maintaining the temperature at 50°C. After synthesis, the solution was centrifuged (3700 rpm for 2 min) and to remove excess ammonia water, the product was washed with deionized water until neutral pH was obtained. The reaction product was transferred to a ceramic vessel and dried for 24 hours at 90°C. The dried product was ground to obtain a homogeneous powder. The reaction resulted in a product with a homogeneous structure and spherical morphology (SEM). The obtained product is phase-pure hydroxyapatite (XRD). The FTIR spectrum shows absorption bands characteristic for HA at 3572 cm⁻¹ indicating vibrations of hydroxyl groups on the HA surface. Bands in the range 3237-3422 cm⁻¹ and 1634 cm⁻¹ are related to the presence of water. Bands at 1453 cm⁻¹ , 1421 cm⁻¹ and at 874 cm⁻¹ are responsible for C-O bonds in carbonate groups CO₃²⁻. The bands corresponding to the vibrational vibrations associated with phosphate ions were identified at 962 cm⁻¹, 468cm⁻¹, 1092 1033 cm⁻¹, 603 562 cm⁻¹.

Hydroxyapatite (HA) is then subjected to a two-stage surface modification using the silane precursor 3-aminopropyltriethoxyilane (APTES) and pyomelanin. In the first stage, a weighed amount of 6.00 g HA and 30 mL of anhydrous toluene are placed in the flask. The flask with the HA suspension is placed in an ultrasonic bath and sonicated 3 times for 3 minutes. The whole is then sonicated for 5 minutes at an amplitude of 50%. The HA particle suspension is then mixed using a magnetic stirrer at 120°C for 10 minutes (350 rpm). After this time, 3 mL of APTES is added to the suspension and the reaction is continued for 24 hours with a reflux condenser. The HA-APTES suspension is then centrifuged in a laboratory centrifuge (3000 rpm, 5 minutes) and washed three times with anhydrous toluene. The obtained precipitate is transferred to a Petri dish and dried in a vacuum dryer at 80°C for 24 hours. The presence of 3-aminopropyltriethoxysilane on the hydroxyapatite surface was confirmed by Fourier transform spectroscopic method (FTIR-ATR). The spectrum of the HA-APTES product shows absorption bands at 3570 cm⁻¹ indicating vibrations of hydroxyl groups on the HA surface. Characteristic bands in the range 1483-1420 cm⁻¹ and at 875 cm⁻¹ are responsible for C-O bonds in carbonate groups CO₃²⁻. The presence of weakly intense bands at wave numbers 2960, 2932 and 2858 cm⁻¹ is responsible for the vibrations of the CH bonds in the alkyl groups of APTES (CH₂), which confirms the structure of the first-stage product. Quantitative analysis of APTES content on the HA surface after the first modification stage was performed using the thermogravimetric method. Based on the thermogram measured in a nitrogen atmosphere, the total mass loss in the temperature range of 25 - 900°C is determined, which amounts to 10.82% by weight for the product. The actual APTES content on the HA surface, calculated from the difference between the total mass loss in the range of 25 - 900°C for HA-APTES and for pure HA, is 4.03 wt%. Thermal stability temperature of the product corresponding to the temperature of 5% mass loss (T_{-5%}) is 378.4°C. The modified HA-APTES particles are modified with pyomelanin (PyoMₛₒₗ). In order to activate the carboxyl groups of PyoMₛₒₗ, 179 mg of PyoMₛₒₗ, 229 mg of EDC and 418 mg of sulfo-NHS and 10 mL of MES buffer (pH=6) are placed in a round-bottom flask and the whole mixture is mixed on a magnetic stirrer for 30 min. After this time, the modified HA particles (HA-APTES) are dispersed in 20 mL of MES buffer (pH=6), sonicated with an amplitude of 50% and added to the mixture of PyoMₛₒₗ, EDC and NHS. The mixture is mixed using a magnetic stirrer for 24 hours. The suspension is then centrifuged to isolate the modified HA-APTES-PyoMₛₒₗ particles, washed 3 times with MES buffer (pH=6) and dried under vacuum at 70°C for 24 hours. PyoMₛₒₗ content on the surface of HA-APTES particles was calculated based on thermogravimetric analysis and is 2.58 wt%. The thermal stability of the product corresponding to the temperature of 5% mass loss is 277.0°C, which is higher than the thermal stability of PyoMsol (T_{-5%}=81.0°C). Presence of PyoMₛₒₗ covalently attached to APTES-modified HA particles was confirmed by FTIR-ATR analysis. On the spectrum of the product HA-APTES-PyoMₛₒₗ a characteristic band is observed at 1736 cm⁻¹, which indicates the first amide band originating from the vibrations of the C=O group and proves the covalent attachment of the carboxyl group of PyoM to the free amino groups of APTES on the HA surface.

Cytocompatibility and activation of the nuclear factor NF-κB HA-APTES-PyoM were tested analogously to Example 1. HA-APTES-PyoM demonstrated biological safety according to the ISO 10993-5:2009 standard for both tested cell lines in the concentration range of 1-100 µg/mL and activated the NF-κB pathway in the concentration range of 100-1000 µg/mL. Biological studies have shown that HA-APTES-PyoM *in situ* is cytocompatible and has beneficial immunomodulatory properties in the context of activating the NF-κB pathway.

### Example 6

### Synthesis of nHA1200°C calcined nanohydroxyapatite

Calcined hydroxyapatite (nHA1200°C), which is one of the components of the osteogenic composites being the subject of the invention, was obtained in accordance with Example 2 of patent application number P.442461. The first stage of synthesis was analogous to the synthesis of HA in Example 2 above, and then after drying the obtained HA, the product was ground and subjected to the calcination process at 1200°C for 1 hour, in accordance with the calcination conditions: heating 10°C/min to 1200°C, maintaining the temperature of 1200°C for 1 hour, cooling 10°C/min to 20°C, air cooling at room temperature. After calcination, the product was ground again to obtain a homogeneous powder.

The reaction resulted in a product with a spherical morphology and a grain size ranging from 692.4 nm to 958.0 nm, which phase consisted of approximately 94% hydroxyapatite and approximately 6% β-calcium phosphate. The FTIR spectrum shows characteristic bands originating from the stretching vibrations of the hydroxyl (OH-) groups (strong band at 3571 cm⁻¹, weak band at 632 cm⁻¹), bands originating from symmetric and asymmetric stretching vibrations of PO bonds in phosphate groups (PO₄³⁻) (1091 cm⁻¹, 1048 cm⁻¹, 961 cm⁻¹) and bands originating from bending vibrations of OPO bonds of phosphate groups (PO₄³⁻) (601 cm⁻¹, 570 cm⁻¹, 438 cm⁻¹). A broad band at 3434 cm⁻¹ is also visible, derived from adsorbed water.

### Example 7

### Synthesis of poly(glycerol adipate) - PGA

25.00 g of divinyl adipinate, 11.61 g of glycerol, and 1.10 g of Novozyme 435 were weighed into a 250 mL round-necked double-necked flask. 50 mL of anhydrous tetrahydrofuran was added and stirred on a magnetic stirrer until the divinyl adipate was dissolved. The freezing cycle in liquid nitrogen and vacuum was then repeated three times on a vacuum-nitrogen Schlenk line. Then it was stirred (250 rpm) and heated at 40°C under a Schlenk reflux condenser under a nitrogen atmosphere for 24 hours. After 24 hours, the Novozym 435 enzyme was filtered from the reaction mixture on a glass filter. The solvent was evaporated from the filtrate on a rotary evaporator. The product was dried at 95°C for 1 hour to deactivate any enzyme residue. The product was then dried in a vacuum oven for 3 days at 40°C. The product was purified by dialysis of a solution of the reaction product in acetone on a 1kD MWCO membrane. Dialysis was performed for 48 hours, and the acetone was changed after 24 hours. The solvent was evaporated from the dialysis solution on a rotary evaporator. The product was dried in a vacuum dryer at 40°C for 24 hours. The product was obtained in the form of an oily, colorless, high-viscosity liquid with a yield of 75%. The structure of the product was confirmed by proton and carbon magnetic resonance (¹H NMR and ¹³C NMR), Fourier transform infrared spectroscopy (FTIR).

¹H NMR (aceton-d₆, 600MHz), δ (ppm): 5.29 (t,1H), 5.09 (m, 1H), 4.89 (1H, m), 4.37 (d, 2H), 4.35 (d, 1H), 4.13 (m, 2H), 4.11 (s, 1H), 4.06 (d, 1H), 3.84 (t, 1H), 3.70 (d, 1H) 3.56 (m, 1H), 2.91 (s, 1H), 2.38 (s, 4H, -CH₂C=O), 1.65 (s, 4H, -HC₂-CH₂-).

¹³C NMR (aceton-d₆, 600MHz), δ (ppm): 75.47 (s), 72.20 (s), 69.95 (s), 69.11 (s), 67.22 (s), 65.39 (s), 64.96 (s), 63.65 (s), 63.15 (s), 62.35 (s), 61.98 (s), 60.79 (s), 60.42 (s).

FTIR-ATR: The FTIR-ATR spectrum shows bands at 3459 cm⁻¹ and 1417 cm⁻¹ derived from stretching and bending vibrations of -OH groups. Bands at 2950 cm⁻¹, 2874 cm⁻¹, 1455 cm⁻¹ characterize the stretching and bending vibrations of the C-H bonds located in the PGA main chain. High intensity band at 1728 cm⁻¹ comes from the stretching vibrations of the carbonyl group (C=O). Bands in the range 1300-1050 cm⁻¹ characterize the stretching and deformation vibrations of COC bonds.

### Example 8

### Obtaining the biocomposite PGA_nHA1200_nHA-APTES-PyoM

10.00 g of poly(glycerol adipate) was dissolved in 62.01 mL of dry dioxane in a 100 mL round-bottom flask. The solution was stirred at 50°C on a magnetic stirrer (250 rpm) under reflux. 5.34 mL of L-lysine diisocyanate was dropped into the solution, stirring of the reaction system was continued for 5 h. w temp. 50°C under nitrogen atmosphere.

To prepare one foam, 1.0 g of NaCl with a grain size of 400-500 µm, 100 mg of nHA1200°C and 2 mg of nHA-APTES-PyoM were mixed. The previously prepared mixture with a total mass of 1.102 g was transferred to the wells of a 36-well silicone plate and each well was filled with 500 µL of the PGA and L-lysine diisocyanate reaction solution. The prepared plate was left in air for 12 hours, then frozen at -20°C for 24 hours and lyophilized for another 24 hours.

After lyophilization, the plates were heated at 60°C for 48 hours. In the next stage, sodium chloride was removed by placing the foams in a beaker with deionized water - the salt rinsing process was carried out on a magnetic stirrer. The water was changed after 30 minutes, 6 hours and 24 hours. The resulting materials are in the form of foams PGA _nHA1200_nHA-APTES-PyoM was dried at 50°C for 24 h and characterized using DSC, TGA and DMA.

The glass transition temperature of the polymer in the PGA_nHA1200_nHA-APTES-PyoM composite determined from the DSC curve measured during the first heating of the sample at a rate of 10°C/min in a nitrogen flow of 60 mL/min is 8.0°C. The thermal stability temperature of the polymer corresponding to the temperature of 10% mass loss, determined from the thermogravimetric curve measured at a heating rate of 10°C/min in a nitrogen flow of 60 mL/min, is 331.2°C.

The compressive storage modulus and compressive loss modulus of the composite in the form of cylinders with a diameter of 15.0 mm and a height of 6.5 mm determined at a temperature of 37°C, a frequency of 10 Hz and a strain of 20 µm are 29.967 MPa and 16.605 MPa, respectively.

### Example 9

### Obtaining the biocomposite PGA_nHA1200_nHA-PyoM_{in situ}

10.00 g of poly(glycerol adipate) was dissolved in 62.01 mL of dry dioxane in a 100 mL round-bottom flask. The solution was stirred at 50°C on a magnetic stirrer (250 rpm) under reflux. 5.34 mL of L-lysine diisocyanate was dropped into the solution, stirring of the reaction system was continued for 5 h. w temp. 50°C under nitrogen atmosphere.

In a separate beaker, 1.0 g of NaCl with a grain size of 400-500 µm, 100 mg of nHA 1200°C and 2 mg of nHA-PyoM*_{in situ}* were mixed. The previously prepared mixture with a total mass of 1.102 g was transferred to the wells of a 36-well silicone plate and each well was filled with 500 µL of the PGA and L-lysine diisocyanate reaction solution. The prepared plate was left in air for 12 hours, then frozen at -20°C for 24 hours and lyophilized for another 24 hours. After lyophilization, the plates were heated at 60°C for 48 hours. In the next stage, sodium chloride was removed by placing the foams in a beaker with deionized water - the salt rinsing process was carried out on a magnetic stirrer. The water was changed after 30 min, 6 and 24 h. The obtained materials in the form of PGA _nHA1200_nHA-PyoM*_{in situ}* foams were dried at 50°C for 24 h and characterized using DSC, TGA and DMA techniques.

The glass transition temperature of the polymer in the PGA_nHA1200_nHA-PyoM*_{in situ}* composite determined from the DSC curve measured during the first heating of the sample at a rate of 10°C/min in a nitrogen flow of 60 mL/min is 7.4°C. The thermal stability temperature of the polymer corresponding to the temperature of 10% mass loss, determined from the thermogravimetric curve measured at a heating rate of 10°C/min in a nitrogen flow of 60 mL/min, is 327.8°C.

The compressive storage modulus and compressive loss modulus of the composite in the form of cylinders with a diameter of 15.0 mm and a height of 6.5 mm determined at a temperature of 37°C, a frequency of 10 Hz and a strain of 20 µm are 18.433 MPa and 14.094 MPa, respectively.

### Example 10

### Confirmation of cytocompatibility and activation of the NF-κB pathway by PGA _nHA1200_nHA-PyoM_{in situ}

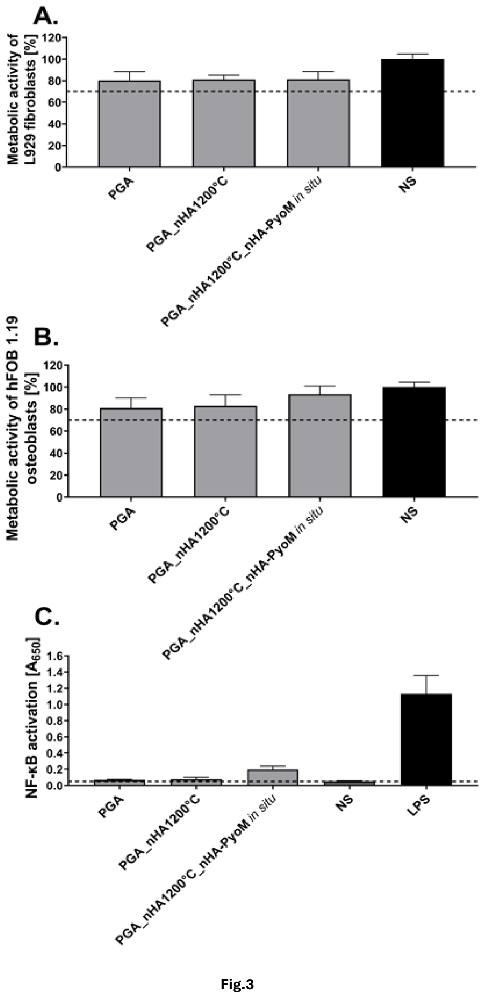

Cytocompatibility of PGA _nHA1200_nHA-PyoM*_{in situ}* was tested in the MTT tetrazolium salt reduction test according to ISO 10993-5:2009 on the *in vitro* model and activation of the nuclear factor NF-κB in the human monocyte reporter model THP1-Blue^{™} NF-κB Cells. Biological activity of the PGA _nHA1200_nHA-PyoM*_{in situ}* biocomposite was shown in Fig. 3 in terms of cytocompatibility (A) with reference L-929 fibroblasts and (B) hFOB 1.19 osteoblasts and (C) activation of the NF-κB pathway.

PGA _nHA1200_nHA-PyoM*_{in situ}* showed biological safety according to the ISO 10993-5:2009 standard for both tested cell lines and activated the NF-κB pathway, which was not observed in the case of the control composites (PGA and PGA_nHA1200). Biological studies have shown that PGA _nHA1200_nHA-PyoM*_{in situ}* is cytocompatible and has beneficial immunomodulatory properties in the context of activating the NF-κB pathway.

### Example 11

### Confirmation of cytocompatibility and activation of the NF-κB pathway by PGA__nHA1200_nHA-APTES-PyoM

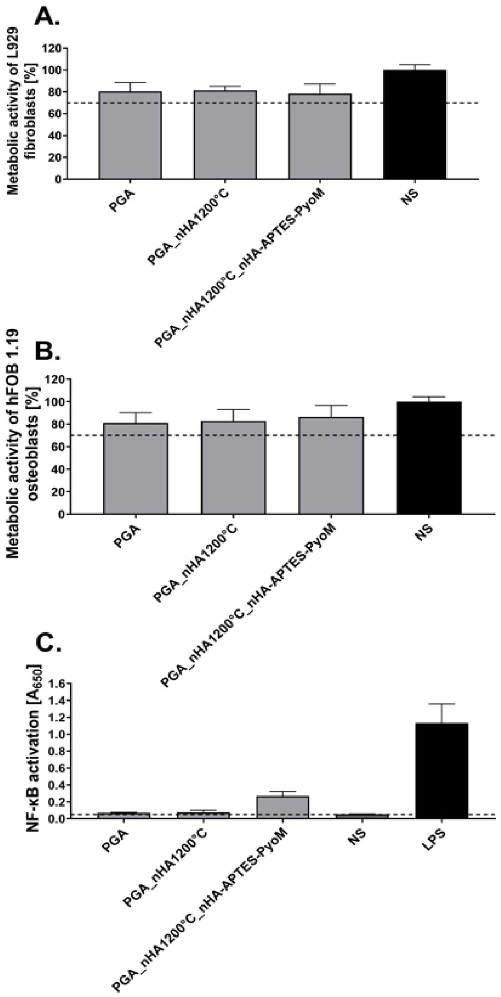

Cytocompatibility and activation of the nuclear factor NF-κB by PGA_nHA1200_nHA-APTES-PyoM were tested analogously to Example 10. The biological activity of the PGA_nHA1200_nHA-APTES-PyoM biocomposite is shown in Fig. 4 in terms of cytocompatibility (A) with reference L-929 fibroblasts and (B) hFOB 1.19 osteoblasts and (C) activation of the NF-κB pathway. PGA_nHA1200_nHA-APTES-PyoM demonstrated biosafety according to ISO 10993-5:2009 for both tested cell lines and activated the NF-κB pathway, which was not observed for the control composites (PGA and PGA_nHA1200). Biological studies have shown that PGA_nHA1200_nHA-APTES-PyoM is cytocompatible and has beneficial immunomodulatory properties in the context of NF-κB pathway activation.

### Example 12

### Cell proliferation, alkaline phosphatase activation and osteoinductive properties of PGA _nHA1200_nHA-PyoM_{in situ}

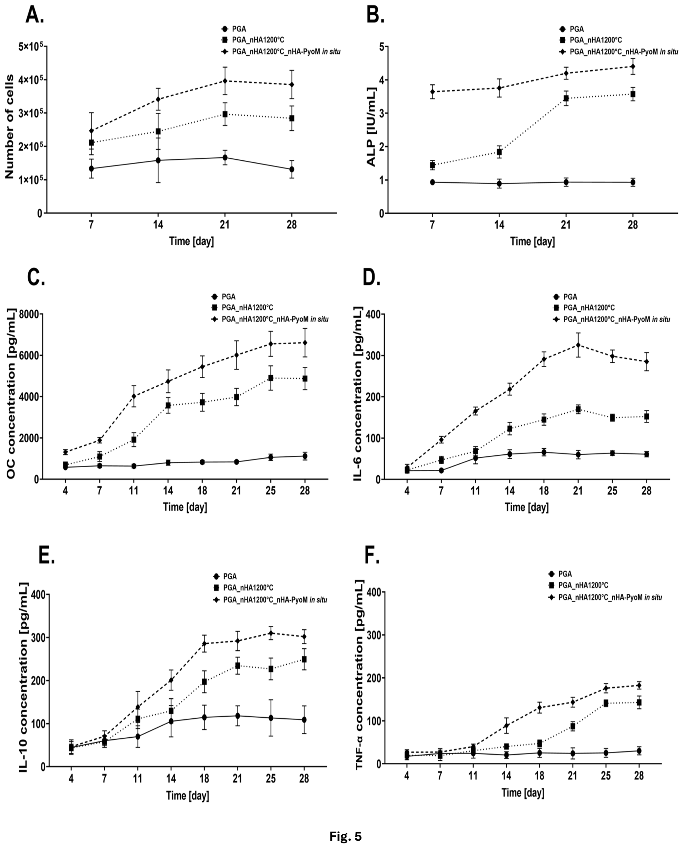

The proliferation of hFOB 1.19 osteoblasts was assessed using the CyQuant^{™} assay, alkaline phosphatase (ALP) activity was assessed using the p-NPP (para-nitrophenylphosphate) hydrolysis assay, and osteoinductive properties were verified by assessing the secretion level of cytokines (osteocalcin [OC], interleukin [IL]-6, IL-10, and tumour necrosis factor [TNF]-α) using an enzyme-linked immunosorbent assay (ELISA). Biological activity of the PGA _nHA1200_nHA-PyoM*_{in situ}* biocomposite was shown in Fig. 5 in terms of proliferation (A), ALP activity (B), secretion: OC (C), IL-6 (D), IL-10 (E) i TNF-α (F).

PGA _nHA1200_nHA-PyoM*_{in situ}* indeed increased the number of hFOB 1.19 osteoblasts, ALP activity and secretion of osteoinductive cytokines (OC, IL-6, IL-10, TNF-α), compared to the control composites (PGA and PGA_nHA1200). Biological studies have shown that PGA_nHA1200_nHA-PyoM*_{in situ}* favourably supports osteoblast proliferation, increases the activity of pro-osteoinductive ALP and has beneficial osteoinductive properties.

### Example 13

### Cell proliferation, alkaline phosphatase activation and osteoinductive properties of PGA_nHA1200_nHA-APTES-PyoM

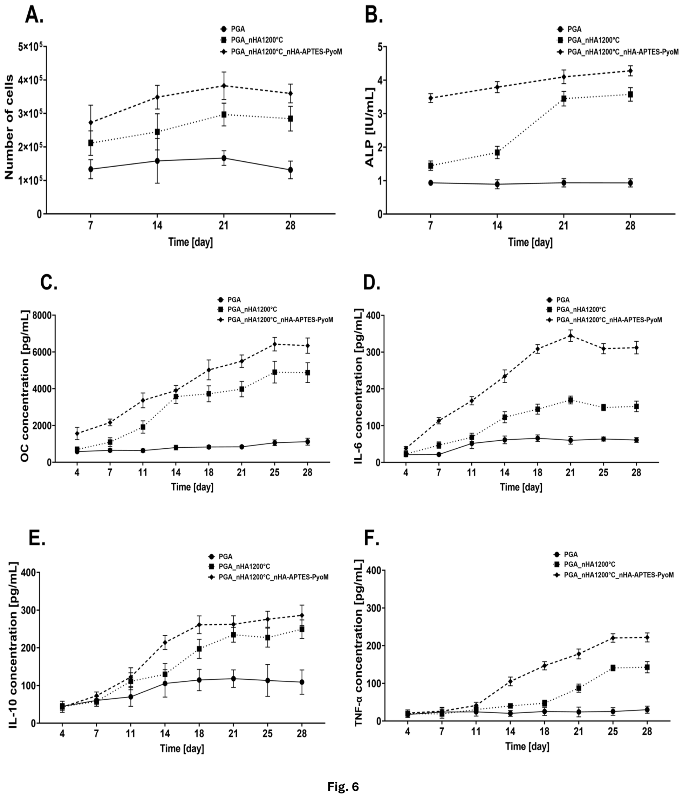

The hFOB 1.19 osteoblast proliferation, alkaline phosphatase (ALP) activity and osteoinductive properties were examined analogously to Example 9. The biological activity of the PGA_nHA1200_nHA-APTES-PyoM biocomposite is shown in Fig. 6 in terms of proliferation (A), ALP activity (B), secretion: OC (C), IL-6 (D), IL-10 (E) i TNF-α (F). PGA _nHA1200_nHA-PyoM*_{in situ}* indeed increased the number of hFOB 1.19 osteoblasts, ALP activity and secretion of osteoinductive cytokines (OC, IL-6, IL-10, TNF-α), compared to the control composites (PGA and PGA_nHA1200). Biological studies have shown that PGA_nHA1200_nHA-APTES-PyoM beneficially supports osteoblast proliferation, increases the activity of pro-osteoinductive ALP and has beneficial osteoinductive properties.

### Example 14

### Confirmation of the antibacterial activity of PGA_nHA1200_nHA-PyoM_{in situ}

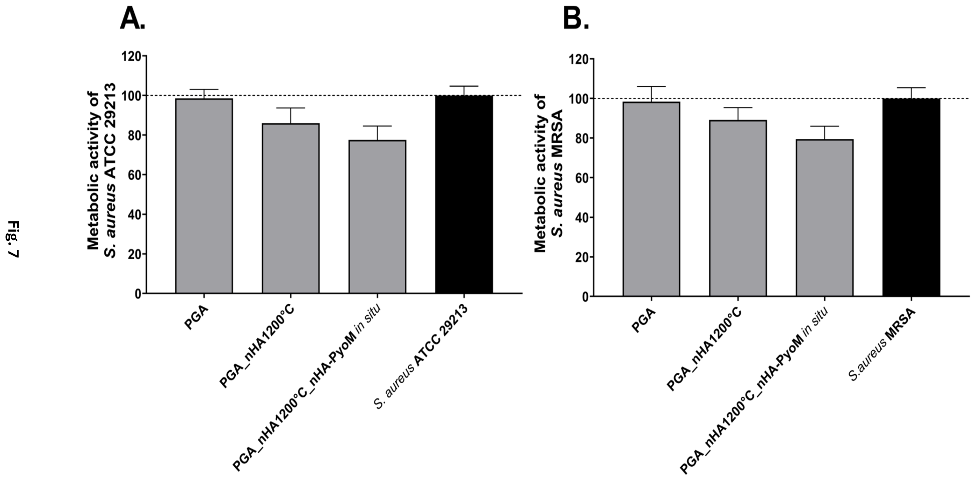

Antibacterial properties of PGA_nHA1200_nHA-PyoM*_{in situ}* against reference and clinical Staphylococcus aureus was tested in the resazurin reduction test. Biological activity of the PGA _nHA1200_nHA-PyoM*_{in situ}* biocomposite was shown in Fig. 7 in terms of limiting the metabolic activity of the reference strain *Staphylococcus aureus* ATC 29213 (A) and a clinical methicillin-resistant strain isolated from bone tissue infected with *Staphylococcus aureus* MRSA (B). PGA_nHA1200_nHA-PyoM*_{in situ}* significantly limited the metabolic activity of both tested staphylococcal strains, compared to the control composites (PGA and PGA_nHA1200). Biological studies have shown that PGA _nHA1200_nHA-PyoM*_{in situ}* shows favourably antibacterial action against staphylococci causing bone tissue infections.

### Example 15

### Confirmation of the antibacterial activity of PGA_nHA1200_nHA-APTES-PyoM

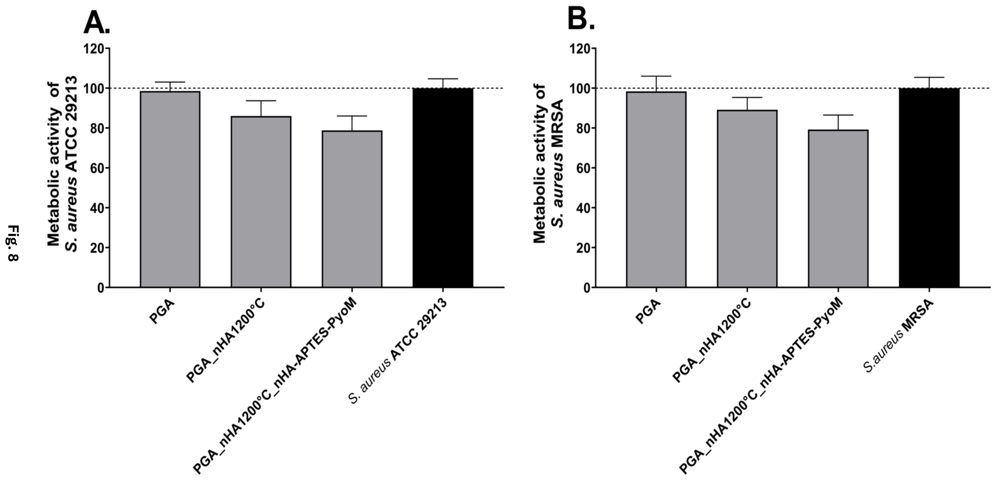

The antibacterial properties of PGA__nHA1200_nHA-APTES-PyoM against reference and clinical Staphylococcus aureus were tested in the resazurin reduction assay. The biological activity of the PGA_nHA1200_nHA-APTES-PyoM biocomposite is shown in Fig. 8 in terms of limiting the metabolic activity of the reference strain *Staphylococcus aureus* ATC 29213 (A) and a clinical methicillin-resistant strain isolated from bone tissue infected with *Staphylococcus aureus* MRSA (B). PGA_nHA1200_nHA-APTES-PyoM significantly limited the metabolic activity of both tested staphylococcal strains, compared to the control composites (PGA and PGA_nHA1200). Biological studies have shown that PGA_nHA1200_nHA-APTES-PyoM exhibits favourably antibacterial action against staphylococci causing bone tissue infections.

### Example 16

### Exclusion of pro-inflammatory properties of PGA_nHA1200_nHA-PyoM_{in situ} and PGA_nHA1200_nHA-APTES-PyoM

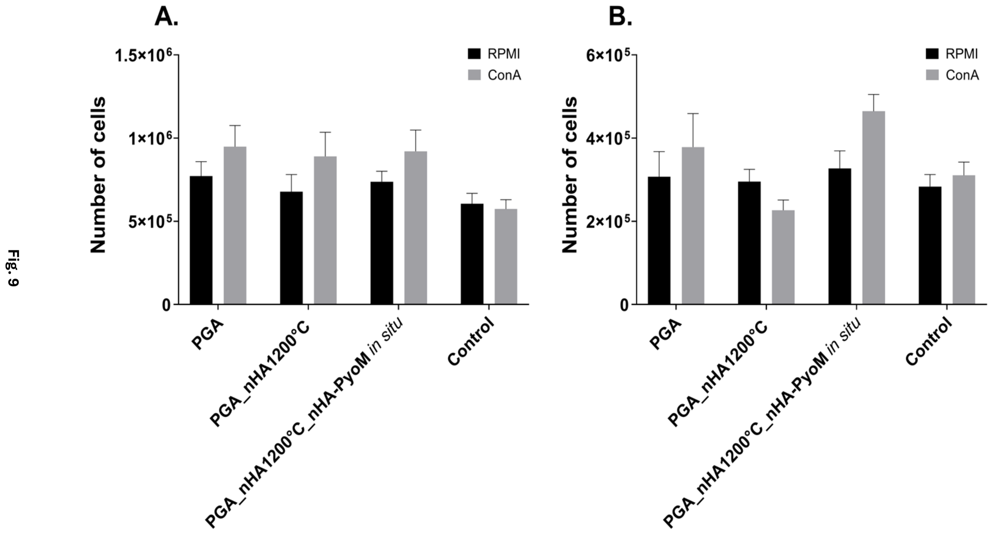

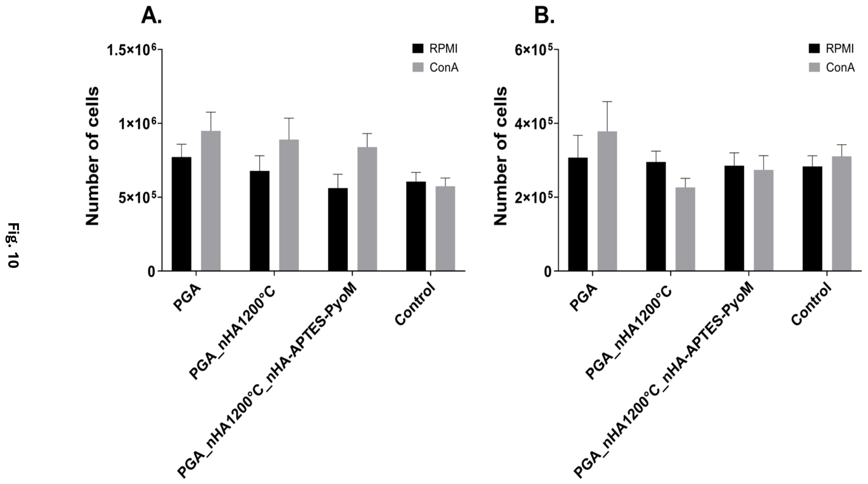

Pro-inflammatory properties of PGA_nHA1200_nHA-PyoM*_{in situ}* and PGA_nHA1200_nHA-APTES-PyoM were evaluated in the *in vivo* system in the study of the generalized inflammatory response in a mouse model. Proliferation of lymphocytes isolated from the spleens and lymph nodes of mice that were subcutaneously administered aqueous extracts obtained after incubation with the composites: PGA _nHA1200_nHA-PyoM*_{in situ}* and PGA_nHA1200_nHA-APTES-PyoM, shown in Fig. 9 and Fig. 10 (A) - spleen, (B) - lymph nodes.

The results obtained in this study indicate that lymphocytes isolated from the spleens (A) and lymph nodes (B) of animals exposed to PGA nHA1200_nHA-PyoM*_{in situ}* and PGA_nHA1200_nHA-APTES-PyoM proliferate spontaneously in medium alone and/or in the presence of concanavalin A - Con A (a proliferation stimulator) with a similar intensity as cells from animals exposed to the control composites (PGA and PGA_nHA1200). *In vivo* studies showed that the tested composites do not contain any potentially irritating or pro-inflammatory substances that enter the solution and cause general inflammation in the body.

### Bibliography:

1. Mohd Puad, N. A. S.; Abdul Haq, R. H.; Mohd Noh, H.; Abdullah, H. Z.; Idris, M. I.; Lee, T. C. Synthesis Method of Hydroxyapatite: A Review. Materials Today: Proceedings. 2019, 29, 233-239. https://doi.org/10.1016/j.matpr.2020.05.536.
2. Urbaniak, M. M.; Gazińska, M.; Rudnicka, K.; P 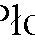 ociński, P.; Nowak, M.; Chmiela, M. In Vitro and In Vivo Biocompatibility of Natural and Synthetic Pseudomonas aeruginosa Pyomelanin for Potential Biomedical Applications. Int. J. Mol. Sci. 2023, 24 (9). https://doi.org/10.3390/ijms24097846.
3. Lorquin, F.; Ziarelli, F.; Amouric, A.; Di Giorgio, C.; Robin, M.; Piccerelle, P.; Lorquin, J. Production and Properties of Non-Cytotoxic Pyomelanin by Laccase and Comparison to Bacterial and Synthetic Pigments. Sci. Rep. 2021, 11 (1). https://doi.org/10.1038/s41598-021-87328-2.
4. Yang, N.; Liu, Y. The Role of the Immune Microenvironment in Bone Regeneration. International Journal of Medical Sciences. Publisher 2021, pp 3697-3707. https://doi.org/10.7150/IJMS.61080.
5. Xu, J.; Yu, L.; Liu, F.; Wan, L.; Deng, Z. The Effect of Cytokines on Osteoblasts and Osteoclasts in Bone Remodeling in Osteoporosis: A Review. Frontiers in Immunology. 2023. https://doi.org/10.3389/fimmu.2023.1222129.
6. Urbaniak, M. M.; Rudnicka, K.; Gościniak, G.; Chmiela, M. Can Pyomelanin Produced by Pseudomonas aeruginosa Promote the Regeneration of Gastric Epithelial Cells and Enhance Helicobacter pylori Phagocytosis? Int. J. Mol. Sci. 2023, 24 (18), 13911. https://doi.org/10.3390/ijms241813911.
7. Liu, D.; Zhong, Z.; Karin, M. NF-KB: A Double-Edged Sword Controlling Inflammation. Biomedicines. 1, 2022. https://doi.org/10.3390/biomedicines10061250.
8. Furlani, F.; Pota, G.; Rossi, A.; Luciani, G.; Campodoni, E.; Mocerino, F.; D'Errico, G.; Pezzella, A.; Panseri, S.; Vitiello, G.; Sandri, M. Designing Bioinspired Multifunctional Nanoplatforms to Support Wound Healing and Skin Regeneration: Mg-Hydroxyapatite Meets Melanins. Colloids Surf. B Biointerfaces. 2024, 235, 113756. https://doi.org/10.1016/j.colsurfb.2024.113756.
9. Szterner, P.; Biernat, M. The Synthesis of Hydroxyapatite by Hydrothermal Process with Calcium Lactate Pentahydrate: The Effect of Reagent Concentrations, pH, Temperature, and Pressure. Bioinorg. Chem. Appl. 2022, 2022 (1). https://doi.org/10.1155/2022/3481677.
10. Tang, G.; Liu, Z.; Liu, Y.; Yu, J.; Wang, X.; Tan, Z.; Ye, X. Recent Trends in the Development of Bone Regenerative Biomaterials. Frontiers in Cell and Developmental Biology. Frontiers Media S.A. May 7, 2021. https://doi.org/10.3389/fcell.2021.665813.
11. Bolamperti, S.; Villa, I.; Rubinacci, A. Bone Remodeling: An Operational Process Ensuring Survival and Bone Mechanical Competence. Bone Research. Springer Nature December 1, 2022. https://doi.org/10.1038/s41413-022-00219-8.
12. Florencio-Silva, R.; Sasso, G. R. D. S.; Sasso-Cerri, E.; Simões, M. J.; Cerri, P. S. Biology of Bone Tissue: Structure, Function, and Factors That Influence Bone Cells. BioMed Research International. Hindawi Publishing Corporation 2015. https://doi.org/10.1155/2015/421746.

## Claims

1. Pyomelanin-modified hydroxyapatite, **characterized in that** the hydroxyapatite surface is chemically modified by pyomelanin molecules covalently attached via a silane precursor or by pyomelanin molecules embedded between the hydroxyapatite crystal lattice in an *in situ* reaction.

2. A method for producing hydroxyapatite modified with covalently attached pyomelanin, **characterized in that** hydroxyapatite particles are subjected to a two-stage chemical modification, such that in the first stage the particles are reacted with a silane precursor and then the product from the first modification stage is reacted with pyomelanin in a solvent.

3. The method for producing hydroxyapatite modified with covalently attached pyomelanin according to claim 2, **characterized in that** the first stage of modification of hydroxyapatite particles is carried out in a suspension of hydroxyapatite particles at a concentration of 0.1 to 3 g per 5 mL of solvent at a temperature of 100°C to 150°C, during 24 hours, at a mass ratio of hydroxyapatite particles to the volume of the silane precursor of 1:0.5, and the pyomelanin modification reaction in the second stage is carried out using 1-ethyl-3-(3-dimethylaminopropyl)carbodiimide hydrochloride (EDC) as a coupling agent and sodium salt of N-hydroxysulfosuccinimide (sulo-NHS) as an agent increasing the reaction efficiency.

4. The method for producing hydroxyapatite according to any one of the preceding claims, **characterized in that** the solvent is anhydrous toluene, the silane precursor is 3-aminopropyltriethoxylate (APTES), and the pyomelanin is bacterial pyomelanin in a soluble form (PyoMₛₒₗ) or insoluble in water (PyoMᵢₙₛₒₗ).

5. The method for producing hydroxyapatite according to claim 2, **characterized in that** in the first stage, hydroxyapatite particles are subjected to sonication, and the reaction using the EDC coupling agent is carried out in a 4-morpholinoethanesulfonic acid buffer at pH 6.

6. A method for producing hydroxyapatite, wherein pyomelanin molecules are incorporated into the hydroxyapatite crystal lattice in an *in situ* reaction, **characterized in that**
- to the aqueous reaction solution of Ca(OH)₂ pyomelanin is added and then an aqueous solution of orthophosphoric acid is dosed in divided doses,
- the reaction is carried out at a constant temperature in the range of 25°C to 75°C for 2 to 48 hours, at a constant pH of the solution in the range of 9 to 11, under continuous stirring at a speed of 450 rpm to 1600 rpm, and
- the orthophosphoric acid solution is dosed into the reaction mixture over a period of 35-45 minutes, maintaining the equilibrium state of the substrates for no less than 3.5 hours until exhaustion and precipitation of a precipitate with nanometric-sized crystalline particles of hydroxyapatite modified with pyomelanin, after which
- the sediment is purified by repeated centrifugation alternating with washing with deionized water until a neutral pH is obtained,
- it is dried for 24 to 48 hours at a temperature not higher than 90°C.

7. The method for producing hydroxyapatite, wherein pyomelanin molecules are incorporated into the hydroxyapatite crystal lattice in an *in situ* reaction, **characterized in that**
- to the aqueous reaction solution of Ca(OH)₂ the aqueous solution of orthophosphoric acid is dosed in divided doses in the first stage and then
- pyomelanin dissolved or suspended in an ammonia solution is added, wherein the reaction is carried out at a constant temperature in the range of 25°C to 80°C for a time of 2 to 72 hours, at a constant pH of the solution in the range of 9 to 11, and under continuous stirring at a speed of 450 rpm to 1600 rpm, and
- the orthophosphoric acid solution is dosed into the reaction mixture over a period of 35-45 minutes, maintaining the equilibrium state of the substrates for no less than 3.5 hours until exhaustion and precipitation of a precipitate with nanometric-sized crystalline particles of hydroxyapatite modified with pyomelanin, after which
- the sediment is purified by repeated centrifugation alternating with washing with deionized water until a neutral pH is obtained,
- it is dried for 24 to 72 hours at a temperature not higher than 90°C.

8. The method according to claim 7 or 8, **characterized in that** the molar ratio of calcium atoms originating from Ca(OH)₂ to phosphorus atoms originating from H₃PO₄ is Ca/P=1.67, and per 0.05 mole of Ca(OH)₂ there are from 10 to 50 mg of pyomelanin, preferably 25 mg of pyomelanin.

9. Polymer-ceramic biocomposite based on hydroxyapatite, **characterized in that** it comprises poly(glycerol adipate) (PGA) modified with L-lysine diisocyanate ethyl ester, and as a ceramic phase nanohydroxyapatite calcined at 1200°C (nHA1200°C) and hydroxyapatite (HA) modified with pyomelanin (PyoM), obtained by the methods described in any one of claims 2 to 8.

10. The biocomposite according to claim 9, **characterized in that** the pyomelanin-modified hydroxyapatite (HA) (PyoM) is hydroxyapatite with pyomelanin molecules covalently attached via a silane precursor or pyomelanin molecules embedded between the hydroxyapatite crystal lattice by an *in situ* reaction.

11. The biocomposite according to any one of claims 9 to 10, **characterized in that** it consists of consists of poly(glycerol adipate) chemically cross-linked with L-lysine diisocyanate ethyl ester in an amount of 56.65 wt.% based on the total weight of the composite, nanohydroxyapatite calcined at 1200°C in an amount of 42.50 wt.% based on the total weight of the composite, and hydroxyapatite with pyomelanin molecules covalently attached via a silane precursor or pyomelanin molecules incorporated in an *in situ* reaction between the hydroxyapatite crystal lattice in an amount of 0.85 wt.% based on the total weight of the composite, wherein the hydroxyapatite with pyomelanin molecules covalently attached via a silane precursor contains pyomelanin in an amount of 2.58 wt.%. in relation to the total hydroxyapatite modified with pyomelanin, while hydroxyapatite with pyomelanin molecules incorporated in the *in situ* reaction between the hydroxyapatite crystal lattice contains pyomelanin in the amount of 4.29% by weight in relation to the total hydroxyapatite modified with pyomelanin.

12. The biocomposite according to any of the above claims, **characterized in that** it supports osteoblast proliferation and has immunomodulatory, osteoinductive, osteoconductive and antibacterial properties.

13. A method for obtaining a biocomposite with osteoinductive, osteoconductive, immunomodulating and antibacterial properties based on hydroxyapatite and chemically modified poly(glycerol adipate), **characterized in that:**
- in the first stage, chemical modification of the poly(glycerol adipate) prepolymer obtained by a known method is carried out with L-lysine diisocyanate ethyl ester in anhydrous dioxane at a temperature of 20-80°C for 2 to 8 hours;
- then a mixture of previously obtained particles of calcined hydroxyapatite and hydroxyapatite covalently modified with pyomelanin via a silane precursor or particles of calcined hydroxyapatite and hydroxyapatite with pyomelanin particles incorporated in the *in situ* reaction between the crystal lattice of hydroxyapatite and NaCl salt is prepared;
- the mixture is poured with a solution of poly(glycerol adipate) modified with L-lysine diisocyanate ethyl ester in dioxane;
- the resulting dispersion system is frozen at a temperature of -10 to -40 for 12 to 48 hours, preferably -20°C for 24 hours;
- it is then freeze-dried for 12 to 48 hours, preferably 24 hours; after which
- it is crosslinked at temperatures from 30 to 90°C for 12 to 72 hours, preferably 60°C and for 48 hours, and then
- NaCl salt is washed out and dried at 30-80°C for 12-72 hours, preferably 50°C and 24 hours.

14. The method according to claim 13, **characterized in that** the crosslinking agent used in the first stage - L-lysine diisocyanate ethyl ester is used in the amount of 2.64 mmol per 1 g of poly(glycerol adipate), the NaCl salt in the mixture constitutes 90.75% by weight in relation to the weight of the ceramic particle mixture, the chemical modification of poly(glycerol adipate) is carried out at 50°C for 5 hours in a nitrogen atmosphere and sodium chloride with a grain size of 400 to 500 µm is used as the blowing agent.

15. Use of hydroxyapatite and a biocomposite defined in any of the above claims for filling bone defects, bone regeneration and dental, veterinary and orthopedic implantation, including bone oncology.
